# EUROPEAN PATENT APPLICATION

(11) **EP 2 554 057 A1**
(43) Date of publication of application: **06.02.2013**
(21) Application number: 10848788.5
(22) Date of filing: 31.03.2010
(51) Int. Cl.: A23L 1/30, A61K 31/221, A61K 31/4415, A61K 31/51, A61K 31/557, A61K 31/7032, A61K 31/7072, A61P 25/28

(54) **ENTERAL OR ORAL FOOD PRODUCT INTENDED, IN PARTICULAR, FOR NUTRITION AND FOR THE PREVENTION AND IMPROVEMENT OF NEUROLOGICAL ALTERATIONS, NEURODEGENERATIVE ALTERATIONS OR COGNITIVE DISORDERS**

(71) Applicant: Vegenat, S.A., 06011 Badajoz (ES)
(72) Inventor: GIL HERNÁNDEZ, Angel, E-06011 Badajoz (ES); SAN ROMÁN PAIS, Paloma, E-06011 Badajoz (ES); PÉREZ RODRÍGUEZ, Milagros, E-06011 Badajoz (ES)
(74) Representative: Gil-Vega, Victor
(86) International application number: PCT/ES2010/070207
(87) International publication number: WO 2011/121150

(57) **Abstract**

The present invention relates to a complete food product for specific nutritional use, especially intended for enteral or oral nutrition of patients who suffer neurological or neurodegenerative alterations, and for the prevention of cognitive or behaviour disorders, especially in the case of elderly persons, such food product comprising a functional ingredient especially conceived for the nourishment of patients suffering from, or prone to neurological or neurodegenerative alterations, cognitive deterioration or behaviour disorders, as well as a mixture of carbohydrates, a mixture of proteins, a mixture of lipids and dietary fibre, inulin, vitamins and minerals.

## Description

This invention relates to a complete food product, specifically intended for enteral or oral nutrition of patients with neurological or neurodegenerative alterations, and for the prevention of cognitive or behaviour disorders, particularly in the case of elderly persons, wherein such food product comprises a functional ingredient which has been especially conceived for the nutrition of patients suffering from, or prone to the development of neurological or neurodegenerative alterations, cognitive deterioration or behaviour disorders, as well as a mixture of carbohydrates, a mixture of proteins, a mixture of lipids and dietary fibre, inulin, vitamins and minerals.

The complete food product according to the invention is appropriate for nutrition in all those pathological circumstances where there is any kind of obstacle that prevents the satisfaction of the nutritional needs by means of an oral diet, and where a diet which is specially focused on the nutritional treatment or on the prevention of neurological or neurodegenerative alterations, cognitive deterioration or behaviour disorders, is required, especially in the case of elderly patients.

Irrespective of their method of administration, i.e., oral or parenteral, there are many groups in society who must cover their nutrition needs with this kind of products. In this respect, a group which is especially significant is the one comprised by those patients who suffer neurological or neurodegenerative alterations, or elderly individuals who are at risk of suffering such diseases, and in the case of cognitive deterioration or behaviour disorders. In these cases, a perfectly balanced diet, in terms of the nutrients supplied, is required.

In this context, neurodegenerative diseases represent a broad chapter within the field of neurological pathologies. This heading comprises several diseases of unknown origin, which have in common a gradual progress of symptoms, which reflects the gradual disintegration of one or several parts of the nervous system. All of them show some common clinical characteristics, since they have an insidious commencement and progress in a continuous manner, without remissions. Degenerative diseases are classified on the basis of the clinical signs they show and, consequently, it is possible to establish a distinction between those diseases that are characterised by a demential syndrome, being Alzheimer's disease the most clear example of them; those which basically involve movement and posture disorders, such as Parkinson's disease; those which evolve with progressive ataxia, such as the olivopontocerebellar atrophy; those whose basic clinical symptoms are muscle weakness and atrophy, as in the case of amyotrophic lateral sclerosis; and many others which show different symptoms.

These cognitive disorders are the result of an increase of the cellular death processes, which decreases the number of neurones and leads to changes in behaviour. These diseases are mainly related to aging. The increase in life expectancy in our society has entailed an increase of these chronic diseases within the elderly population. The diseases which gradually disintegrate human brain overwhelmingly appear at the old age. Alzheimer's dementia and Parkinson's disease, along with cerebrovascular accidents are among the most frequent causes of physical and mental disability among elderly people.

Alzheimer's disease is the most common cause of dementia, with a prevalence of 26 million people affected around the world. The disease is diagnosed in subjects whose age is around 65, and the prevalence increases with age, the most frequent case being the Late Onset Sporadic Alzheimer's Disease (LOAD). Alzheimer's disease is characterized by a gradual cognitive malfunction and, under the pathogenic point of view, by the accumulation of amyloid plaques at the brain, which could be the result of unbalances between their formation and their clearing or metabolism.

Familial Alzheimer's disease, which represents only 1% of the total number of cases, is an autosomal dominant disease which has been related to mutations of three genes, namely *APP, PSEN1* and *PSEN* 2, that encode the precursor of α-amiloyd and presenelins 1 and 2, respectively. As far as LOAD is concerned, it has been evidenced that the presence of the 4 allele of the apolipoprotein E (APOE) gene increases from 20 to 90% the risk of suffering Alzheimer's Disease and decreases the initial age from 84 to 68 years, and it is currently considered the primary genetic risk in Alzheimer's disease.

Two recent GWA studies, published in 2009, have shed new light on the genetic determining factors of Alzheimer's disease. Both of them confirm that certain gene variants of the *APOE* gene are associated to the disease. Furthermore, a SNP of the *CLU* gene, which is encoded for clusterin, and a block that shows a linkage imbalance, which is found within the gene of the 3b/4b components of the complement component receptor 1 (*CR1*), as well as a SNP of the *PICALM,* gene, which encodes the Phosphatidylinositol binding clathrin assembly protein, are also associated with LOAD.

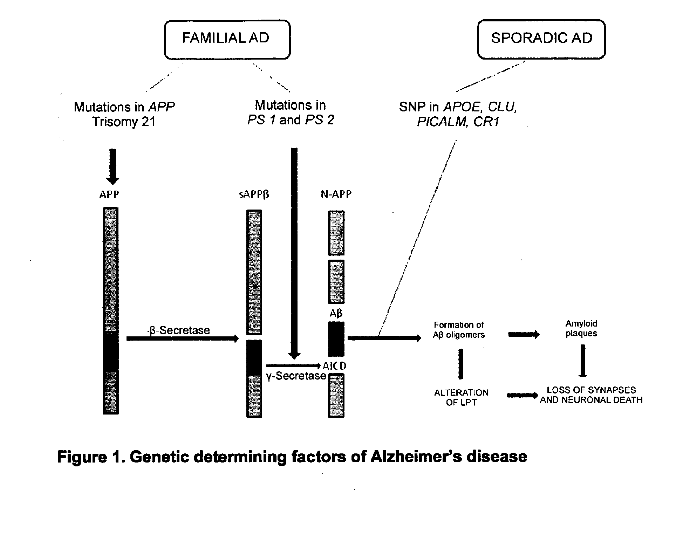

Although the pathogenic mechanism of Alzheimer's has not been completely revealed, the most widely accepted theory is that the accumulation of the amyloid-beta protein (Aβ), an APP processing product, plays a major role in the development of the disease. In this pathway, the APP is initially hydrolysed by α-secretase and, subsequently, by β-secretase, giving rise to different products, including the Aβ peptide. An imbalance between the generation and its metabolism leads to the formation of amyloid plaques that trigger the deterioration of the cognitive development. The Aβ oligomers may inhibit the long-term potentiation at the hippocampus and alter the function of synapses, irrespective of oxidative stress and the inflammation caused by the Aβ aggregated and deposited (refer to Figure 1).

The genes related to the Familial Alzheimer's disease influence the formation of the Aβ protein, while the new variants of the *CLU, CR1* and *PICALM* genes may affect its deterioration. Thus, clusterin shows many similarities with Apoprotein E, and both are present at, and cooperate to the formation of amyloid plaques, and influence on the changes experienced by the clearing pattern of the amyloid protein. On the other hand, the CR1 complement receptor joins phagocytosis-related acceptor molecules, and the over-expression of C3 in transgenic mice entails a lower level of amyloid protein deposits, while its inhibition leads to deposits and neurodegeneration. On the other hand, the product resulting from the *PICALM* gene is involved in the clathrin-mediated endocytosis, which is an essential step for the passing of proteins, lipids, growth factors and neurotransmitters. In tests carried out with cultured cells, the APP is removed from the cell surface by the clathrin and, in case that this latter is of an aberrant nature, the Aβ levels become altered.

Parkinson's disease is an insidious and progressive disease, which causes loss of mobility, trembling, stiffness and alteration of the walking postures. It ranks second among the neurodegenerative syndromes, only after Alzheimer's disease, with an average age of onset of 60 and with an increasing risk with age.

Parkinson's disease is characterized by a neuronal loss in the substantia nigra and other brain regions, and is associated to the formation of intracellular protein aggregates, known as Lewy bodies. One of the main components of these bodies is α-synuclein, a synaptic protein with an unknown function (Gil Hernández A, Olza Meneses J, Gómez Llorente C., Bases genéticas de las enfermedades complejas, In Gil A. (ed). Tratado de Nutrición 2a Ed., Médica Panamericana, Madrid 2010).

From a clinical point of view, dementia is frequently associated to Parkinson's disease and, reciprocally, patients suffering Alzheimer's disease may show symptoms of Parkinson's. From a neuropathological point of view, Lewy bodies often occur simultaneously with the damages typically found in Alzheimer's disease, i.e.. amyloid plaques and neurofibrillary tangles. All this suggest that there is a common pathway in both neurodegenerative diseases where the aggregation of T-protein acts as a common mediator of the toxicity of the amyloid protein and the α-synuclein (Van Es MA, Van der Berg LH., Alzheimer's disease beyond APOE, Nature Genet 2009; 41: 1047-1048).

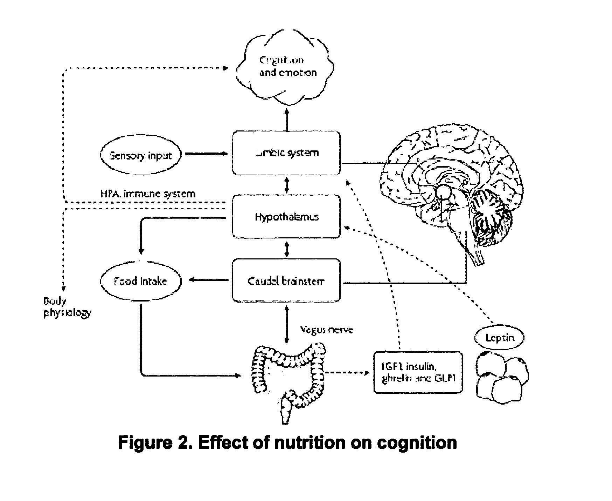

In recent years, researchers have suspected that the relative abundance of specific nutrients might affect cognitive processes and emotions. The study of the influence of the diet factor on neuronal function and synaptic plasticity has revealed some of the vital mechanisms behind the effects of diet on brain health and mental function (refer to Figure 2).

As Fernando Gómez-Pinilla explains in Nature, Brain and Society, "Brain foods: the effects of nutrients on brain function", neuronal circuits involved in the feeding-related behaviour require coordination between the brain centres that regulate energy homeostasis and cognitive functions. The effect of food on knowledge and emotions may even begin before the actual intake, since the sensorial perception of smells and the visual aspect alters the emotional status of brain. The consumption of food entails the release of hormones or peptides, such as insulin and GLP1 (a peptide similar to Type 1 glucagon), to the bloodstream (McNay, E.C., "Insulin and ghrelin: peripheral hormones modulating memory and hippocampal function" Curr. Opin. Pharmacol. 7, 628-632 (2007)). Then these substances may reach specific areas of the brain, like the hypothalamus and the hippocampus, and activate the translation-signal routes that promote synaptic activity and contribute to learning and memory. The chemical messages derived from the adipose tissue through leptin may activate specific receptors of hypothalamus and hippocampus, thus influencing learning and memory. The positive action of leptin on the hippocampus-dependent synaptic plasticity has been well characterized, i.e., its action on the functions of the NMDA (N-methyl-D-aspartate) receptor and its strengthening along the time (Harvey, J. "Leptin regulation of neuronal excitability and cognitive function", Curr. Opin. Pharmacol. 7, 643-647, (2007)). Apart from regulating appetite, the hypothalamus co-ordinates intestinal activity and integrates Visceral Function with structures belonging to the limbic system, such as the hippocampus, the amygdale and the brain cortex. Visceral signals may also modulate knowledge and body physiology through the Hypothalamic-pituitary axis (HPA). The effects on the hypothalamus may also involve the immune system, and certain molecules belonging to such system may affect synaptic plasticity and knowledge. The parasympathetic innervation of the intestine through the vagus nerve provides sensorial information to the brain, thus allowing intestinal activity to influence emotions.

Thus, the role played by certain nutrients and plants in the brain function is being researched, in the light of the growing desire to maintain the intellectual, memory, concentration and learning capacity with, or despite, age. Once the need for glucose as the preferred neurone nutrient has been established, phospholipids and certain antioxidants (vitamins and polyphenols) remain the most deeply studied dietetic components in connection with the optimisation of the brain and cognitive performance. Similarly, studies are being carried out in connection with the neural response to other substances like choline, vitamin complexes (folic acid, B6, B12), Omega-3 fatty acids (DHA), gamma amino-buteric acid (GABA) and other novel products such as Acetyl-L-carnitine or alpha lipoic acid.

U.S. Patent No. US 6.900.180 B1, "Pharmaceutical compositions for alleviating discomfort", relates to products intended for the complete nourishment of kids, sick or elderly people. Such products contain high levels of folic acid, vitamin B6 and vitamin B12, or their functional equivalents. These products improve emotions on healthy children, especially in the case of the younger ones, and they are useful in the treatment and prevention of diseases that are associated with disorders of serotonin and melatonin metabolism. The product described in this patent comprises carbohydrates, fats and proteins, and contains between 44 µg and 4,000 µg of folic acid, between 0.8 µg and 2,000 µg of vitamin B12 and between 50 µg and 10,000 µg of vitamin B6 per every 100 Kcal of such hydrocarbons, fats and proteins, and also contains at least one of riboflavin, thiamine, niacin and zinc.

The international publication WO 2005/000040, "Infant formula", relates to a nutritional supplement comprising an infant milk formula having long chain polyunsaturated fatty acids, sialic acids, and cholesterol, and these compositions can be used to provide enhanced neurological development, gastrointestinal protection, and immune function in both term and preterm infants.

WO 2006/128465 A1, "Phosphatidylserine enriched milk fractions for the formulation of functional foods", relates to a bovine milk derived phosphatidylserine source of natural composition having excellent dispersibility and organoleptic as well as physical stability. The phosphatidylserine source is used in powder or liquid form, as well as in dispersion, and may be derived from buttermilk serum or from microfiltration of serum.

ES 2118629 T3, "Fatty mix containing phospholipids with fatty acids" describes a food formulation which contains a fatty mixture made of oils and/or fats and lecithins comprising long chain polyunsaturated fatty acids from an animal, vegetable or, if appropriate, microbial source, characterised in that the arachidonic acid present in the fatty mixture as phospholipids accounts for 0.2 to 0.3 mg of the total fat and the docosahexaenoic acid present as phospholipids accounts for 0.1 to 2.0 mg of the total fat, and in that both acids present at the fat as triglycerides represent, respectively, 0.005 to 1,5% by weight compared with the total amount of fatty acids present in the mixture.

ES 2158949 T3, "New dietetic compositions based on phospholipids and their use as a nutritional complement" claims a dietetic composition for the feeding of delicate or undernourished patients, characterized in that it contains phospholipids derived from egg yolks, containing from 75 to 80% of phosphatidylcholine and from 15 to 20% of phosphatidylethanolamine, such phospholipids containing essential fatty acids from series n-3 and n-6, associated or mixed with excipients or diluents suitable for alimentary use.

The object of this invention is a complete food product especially intended for the oral or enteral nutrition of patients with neurological or neurodegenerative alterations, and for the prevention of cognitive or behaviour disorders, especially in the case of elderly persons.

In order to clarify the scope and characteristics of the invention, reference will be made to the accompanying figures, wherein:
- Figure 1:: shows a diagram of the determining genetic factors of Alzheimer's disease;
- Figure 2:: shows the vital mechanisms in charge of the action of the diet on brain health and operation;
- Figure 3:: shows a diagram of the cholinergic function;
- Figure 4:: metabolic use of the UMP included in the diet;
- Figure 5:: Functions of choline and of the conversion and metabolic use of choline and phosphatidylcholine;
- Figure 6:: Effect of DHA in the neuroprotection by way of the inhibition of the neural apoptosis.

The Food product which is the object of this invention comprises a functional ingredient which is especially designed for the nutrition of patients suffering from, or who are prone to suffer, neurological or neurodegenerative alterations, cognitive deterioration or behaviour disorders, a mixture of carbohydrates, a mixture of proteins, a mixture of lipids and dietary fibre, inulin, vitamins and minerals, as well as other optional additives or nutritional excipients. The food product of this invention has a perfectly defined nutritional composition and a smoothness and homogeneity which allows it to be conveniently administered through nasogastric tubes, apart from an excellent palatability, which allows an appropriate oral administration.

The mixture of proteins which is present in the food product which is the subject matter of the invention comprises caseinate (50%), vegetable protein (25%), preferably derived from peas, and milk whey proteins (25%) enriched with glycomacropeptide (approximately 0.16 g/100 ml of product), and this mixture of proteins accounts for between 15% and 20% of the total energy supplied by the product.

The mixture of carbohydrates which is present in the food product which is the subject matter of the invention represents between 45 and 50% of the total energy of the product and includes dietary fibre (approx. 2 g /100 ml of product) and inulin (1,2 g/100 ml of product).

The lipidic mixture which is present in the food product which is the subject matter of the invention accounts for 35% of the total energy of the product. The lipidic profile which is present in the food product which is the object of the invention can be summarised as follows:

| | *Compared with 100 g of fat* |
|---|---|
| saturated fatty acids | 20.00% |
| monounsaturated fatty acids | 55.00% |
| polyunsaturated fatty acids | 25.00% |

| | | *Compared with the total fatty acids* |
|---|---|---|
| Saturated fatty acids of which | | 20.00% |
| | MCT | 8.60% |
| monounsaturated fatty acids | | 55.00% |
| polyunsaturated fatty acids of which | | 25.00% |
| | Linoleic acid | 20.41% |
| | Linolenic acid | 2.45% |
| | EPA | 0.47% |
| | DHA | 0.94% |

Although in principle it was considered that the lipids contained in the diet affected the brain through their effects on cardiovascular physiology, they have acquired an increasing importance in the last few years, as far as their direct effects on the brain are concerned. Thus, the omega-3 polyunsaturated fatty acids are among the constituents of cellular membranes and are equally essential for the operation of the brain; it has been generally acknowledged that in the case of mice, a deficiency of these omega-3 fatty acids causes learning and memory disorders. In humans, such deficiency is related to an increasing risk of suffering certain mental disorders, including attention deficit disorder, dyslexia, dementia, depression, bipolar disorder and schizophrenia. Contrary to the beneficial effects of omega-3 rich diets, different epidemiologic surveys indicate that a diet with a high content of *trans* saturated fats adversely affect cognitive capacity (Greenwood, C.E., "High-fat diets, insulin resistance and declining cognitive function", Neurobiol. Aging 26, suppl. 1, 42-45 (2005)). The surveys performed on mice to assess the effects of junk food, with a high content of fats and sugars, have demonstrated a deterioration of cognitive development and a decrease in the synaptic plasticity related to the BDNF levels at the hippocampus only three weeks after the application of such a diet. These surveys suggest that the diet has a direct effect on neurones. However, the worst thing was that this diet increased the neurological load associated to brain damages, which was evidenced through a poorer development of learning tasks and a decrease in the BDNF-mediated synaptic plasticity.

Recent studies may lead to the conclusion that a diet which is rich in omega-3 fatty acids reinforces cognitive processes in humans, and over-regulates certain genes that are important to maintain the synaptic function and plasticity in mice (MCCann, J.C., Am. J. Clin. Nutr. 82, 281-295 (2005)). In turn, those diets which are rich in saturated fatty acids tend to reduce the molecular substrates that support cognitive processes and increase the risk of neuronal malfunction, both in animals and humans. It has been widely recognised that certain intestinal hormones that can access the brain or which are produced by this latter influence on cognitive capacity. Similarly, the widely studied regulators of synaptic plasticity, such as the BDNF, or brain- derived neurotrophic factor, may operate as metabolic modulators, responding to peripheral signals such as the ingestion of foods. Thus, the diet may affect multiple brain processes through the regulation of the routes of neurotransmitters, synaptic transmission, the fluidity of the membrane and the signal transduction pathways.

Thus, in the case of this fatty profile, the linoleic acid/linolenic acid ratio is of approximately 8:1. This is in line with the newest criteria (HWO Report on the Needs of fatty Acids) which recommend a supplement of fatty acids to cover the essential fatty acids requirements. Many other mixtures that are being currently used for enteral nutrition purposes do not contain the appropriate amounts of linolenic acid, in the case of other formulations, the amounts of linolenic acid are excessive and, in many cases, the ratio between both fatty acids is not adequate.

As it has been previously mentioned, omega 3 fatty acids and, especially, docosahexaenoic acid (DHA), are necessary to obtain an optimum neuronal function, as they are among the major constituents of cellular membranes. Omega 3 rich diets are associated to a lower incidence of dementia. Recent reports relate its ingestion to the improvement and maintenance of the cognitive function in healthy individuals, and its important beneficial effects are attributed to the anti-inflammatory capacity of these fatty acids.

On the basis of these considerations, the lipid mixture present in the food product of the invention is supplemented by means of the appropriate amounts of eicosapentaenoic (EPA) and docosahexaenoic (DHA) acid at a rate of 1:2, especially 0,023 g of EPA /100 ml of product and 0,047 g of DHA /100 ml of product.

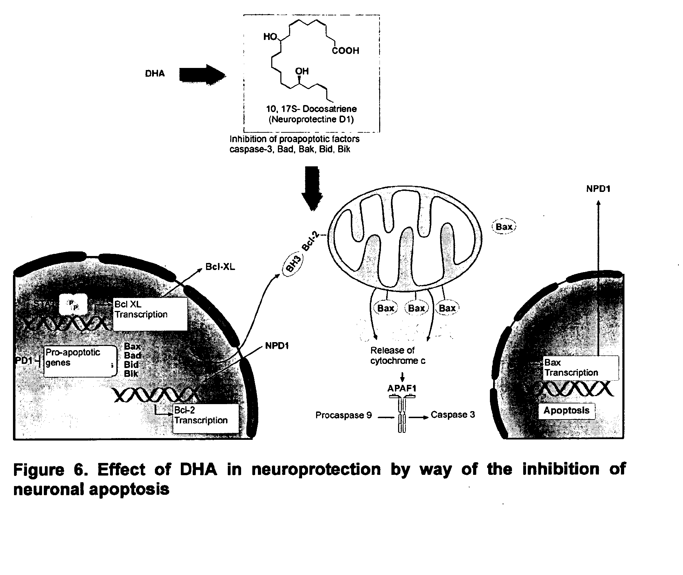

Polyunsaturated fatty acids and, especially EPA and DHA, are precursors of resolvins and protectins. These chemical mediators play an important role in inflammatory processes, for instance, by controlling the duration and the size of the inflammation. It has been recently demonstrated that inflammation plays an important role in many diseases which had been previously considered inflammatory diseases, among them, Alzheimer's disease (refer to "Resolution Phase of Inflammation: novel endogenous anti-inflamatory and proresolving lipid mediators and pathways", Cherles N. Shernan, in Annu. Rev. Inmunol. 2007, 25:101-37) (see figure 6).

The food product which is the object of this invention also comprises a functional ingredient which has been especially designed for the nutrition of patients suffering from, or who are prone to suffer, neurological or neurodegenerative alterations, cognitive deterioration or behaviour disorders. Such functional ingredient also comprises, apart from the previously mentioned amount of DHA, the following components, in the amounts shown:

| | *Per 100 ml of product* |
|---|---|
| UMP (Uridine monophosphate) and/or IMP (inosine monophosphate) | 20 mg |
| Vitamin B1 | 0.1 mg |
| Vitamin B6 | 0.18 mg |
| Vitamin B7 (choline) | 55 mg |
| Vitamin B9 (folic acid) | 40 µg |
| Phospholipids | 39 mg |
| Gangliosides | 2.5 mg |
| Sialic acid | 0.88 mg |

The term "choline", as used herein, refers both to free choline, preferably in salt form, and to Choline bitartrate and choline linked as phosphatidylcholine (Lecithin).

The term "folic acid", as used herein, also includes its anionic form or folate.

In the present description, the terms UMP (Uridine monophosphate) and uridine, as well as IMP and inosine, will be indistinctly used, since the UMP administered orally or enterally, as in the case of the food product of this invention, is quickly degraded and converted into uridine in the digestive tract, and IMP is transformed into inosine.

Phospholipids or phosphatides are among the main constituents of cellular membranes, forming the lipidic layer and serving as resevoirs for the first and second messengers or their precursors, such as acetilcholine, eicosanoides, diacylglicerol and inositol 1,4,5-triphosphate.

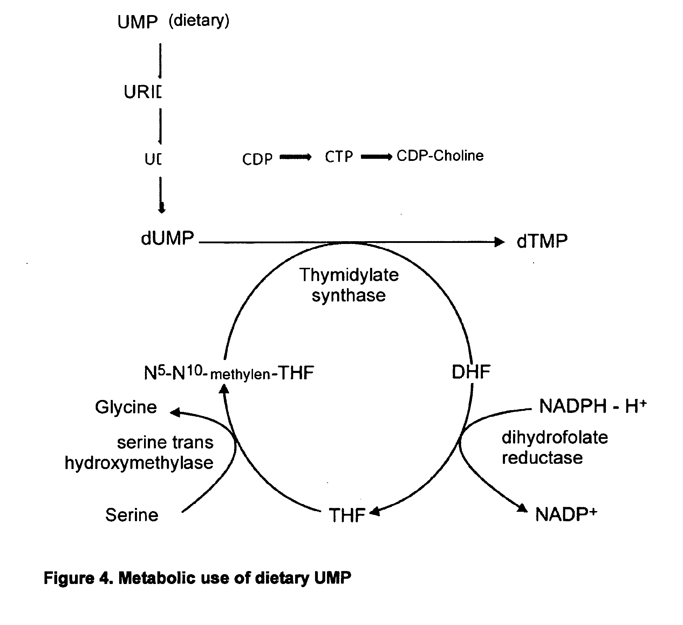

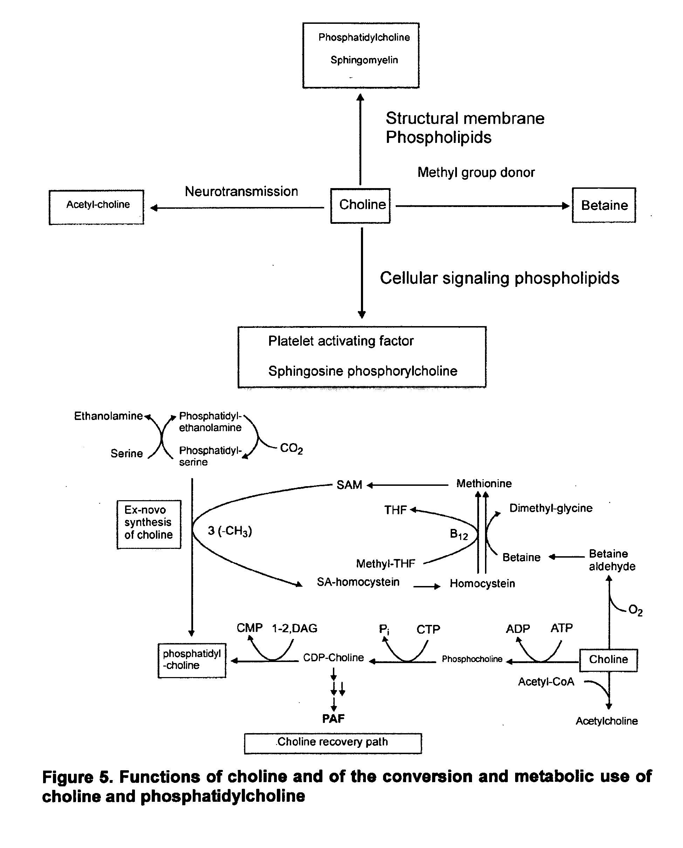

At the brain, the synthesis of the phosphatidylcholine phosphatide may use three circulating precursors, namely choline, a pyrimidine (for instance, uridine, converted into brain phosphatidylcholine through UTP) and a polyunsaturated fatty acid (for instance, DHA). The choline is phosphorylated to form phosphocholine. Uridine is phosphorylated to obtain uridine triphosphate (UMP), which will be subsequently converted into cytidine triphosphate (CTP), a precursor which limits the synthesis speed of phosphatidylcholine. The enzymes that participate in these processes have a low affinity and, consequently, an oral dose of UMP, a source of uridine, entails a sequential increase of brain uridine, UTP and CTP (Cansev and col, 2005). Phosphocholine and CTP are combined to obtain CDP-choline (cytidine-5'-diphosphocholine), which is subsequently combined with diacylglycerol, including species that contain DHA, and obtaining phosphatidylcholine. Richard J. Wurtman and col. ("Synaptic proteins and phospholipids are increased in gerbil brain by administering uridine plus docosahexanoic acid orally", Brain Research 1088 (2006), 83-92) have demonstrated that the oral administration of UMP or DHA, along with a choline - rich diet, may increase the phosphatidylcholine present at the brain, as well as other membrane phosphatides, and the effect perceived with the supplemental administration of DHA and UMP is greater, compared with the separate administration of UMP and DHA. This increase may include the synaptic membranes, since the treatment also increases the levels of pre-synaptic and post-synaptic proteins, influences on theneuronal function and, possibly, on behaviour (Mehmet Cansev and col, "Oral UMP increases brain CDP-coline levels in gerbils", Brain Research 1058 (2005), 101-108) (See figures 4 and 5).

On its part, inosine, acting through a direct intracellular mechanism in cultured neurones, induces the expression of a variety of genes associated to the growth of the axon, including GAP-43, L1 and α-1-tubuline (Benowitz, L.I., Jing, Y., Tabibiazar, R., Jo, S.A., Petrausch, B., Stuermer, C.A., Rosenberg, P.A. & Irwin, N. (1998), J. Biol. Chem. 273, 29626-29634; Petrausch, B., Tabibiazar, R., Roser, T., Jing, Y., Goldman, D., Stuermer, C.A., Irwin, N. & Benowitz, L.I. (2000), J. Neurosci. 20, 8031-8041). The expression of these genes suggests that inosine may act in a similar way *in vivo,* inducing a gene expression program that allows the pyramidal cells of the cortex to extend axon branches, such growth allowing these axons to overcome some of the molecular signals which normally inhibit growth (Chen P, Goldberg DE, Kolb B, Lanser M,Benowitz LI, Inosine induces axonal rewiring and improves behavioral outcome after stroke, Proc. Natl. Acad. Sci. U. S. A. 99 (2002), pp. 9031-9036).

Similarly, to allow the normal operation of brain, an interneuronal communication by means of neurotransmitters is required. The operation of this system entails a delicate balance between those enzymes that produce the neurotransmitters and those which degrade them. One of the main neurotransmitters present at the brain is the acetylcholine, which performs a core role in cognitive functions, such as memory, attention and the behaviour regulating mechanisms. Thus, Alzheimer's disease has been connected with a decrease of acetylcholine and its effects on the brain, known as cholinergic effects. This decrease makes that those patients who suffer the disease experience difficulties to think, to remember and to perform simple works. The cholinergic hypothesis of treatment is based on achieving an increase in the amounts of the acetylcholine neurotransmitter, which is associated o the learning processes and memory, at the intersynaptic space. According to this hypothesis, the origin of Alzheimer's disease could be a deficit of acetylcholine.

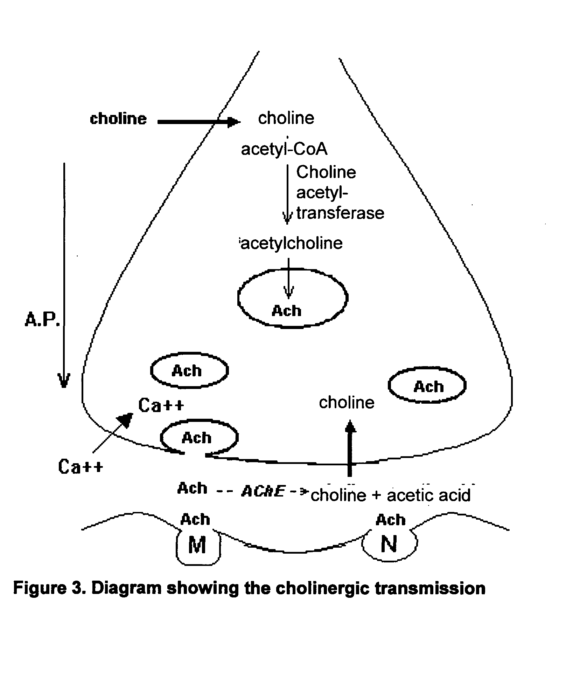

Cholinergic transmission works according to the scheme shown in Figure 3.

Acetylcholine (Ach) is synthesised from choline inside the neurone, through the action of an enzyme called choline acetyltransferase (ChAT). Once it has been synthesised, ACh is stored in vesicles inside the presynaptic neurone. When the nervous pulse occurs, the ACh is released into the intersynaptic space and interacts with the cholinergic receptors of the postsynaptic neurone. These receptors belong to two classes, Nicotinic acetylcholine receptors and Muscarinic acetylcholine receptors, and their interaction with the ACh triggers the nervous signal. The type of muscarinic receptors found at the postsynaptic neurones are those known as M1, while those known as M2 can be found at the presynaptic neurone. The object of these latter ones is to regulate, by means of a feedback mechanism, the ACh concentration within the intersynaptic space. In other words, the interaction of ACh with the M2 receptors indicates that the neurone should not release more neurotransmitters. There is another enzyme in the intersynaptic space, which regulates the concentration of ACh on the basis of its degradation. Acetylcholinesterase (AchE) degrades the neurotransmitter and coverts it into choline and acetyl. Choline captured by the neurone and the synaptic cycle begins once more. The levels of this neurotransmitter may be reestablished through the supply of choline to the diet, preferably in lecithin form (see figure 3).

On the other hand, an adequate level of folate is essential for the brain function, and a deficit of folate may entail a neurological disorder, such as depression or cognitive disability, such deficiency being associated to a number of physiological abnormalities during the development and maturity stages. It has been evidenced that the supplementation of a folate, both by itself and along with other vitamins B, is effective for the prevention of ageing-related cognitive deterioration and dementia, and to reinforce the effect of antidepressants (Mischouolon, D., Raab, M.F., The role of folate in depression and dementia, J. Clin. Psychiatry 68, 28-33 (2007); Corrada, M. and col. "Reduced risk of Alzheimer's disease with high folate intake: The Baltimore Longitudinal Study of Aging", Alzheimer's Dement. 1, A4 (2005); Fioravanti, M. and col. "Low folate levels in the cognitive decline of elderly patients and efficacy of folate as a treatment for improving memory deficits", Arch. Gerontol. Geriatr. 26, 1-13 (1997); Fava M. and col., "Folate, vitamin B12 and homocysteine in major depressive disorder", Am. J. Psychiatry 154, 426-428 (1997)).

Folate is the raw material which intervenes in the metabolism of one-carbon fragments (OCM, from *One Carbon Metabolism),* a process involved in the synthesis and repair of DNA and different methylation reactions. The folate acquired with diet is the only source for the OCM requirements and, therefore, any changes in the intake of folate may influence the balance of intermediate products, among them, the amino acid known as cysteine, known by its toxic effects on cells at high concentrations. It is believed that the alterations of OCM lead to neuronal apoptosis through different ways: (i) by increasing the levels of extracellular homocysteine (Lipton, 1997; Ho et al., 2002); (ii) by altering the methylation process (Ulrey et al., 2005) and (iii) influencing the synthesis of DNA through an unbalance in the ratio of precursors (Fenech, 2001).

Thus, folic acid intervenes as a cofactor in a series of reactions that involve the transfer of a carbon atom, among them, the synthesis of purines and tymidine, i.e., it is essential for the construction of the DNA and RNA components and, on the other hand, it works along with vitamin B12 to help the body to break down and to use and create new proteins. The folate is also responsible for the elimination of homocysteine from blood. It can be also verified that patients suffering from Alzheimer's disease may show high levels of homocysteine and low levels of folate and vitamin B12. In a survey published in The Lancet (2007), performed during a term of three years among 818 individuals of more than 50 years, in connection with short-term memory, mental agility and verbal fluency, an improvement was noticed among those persons who had a daily intake of 800 µg of folic acid.

Gangliosides are glucolipids with very large polar heads formed by units of negatively charged oligosaccharides, which have one or more units of n-acetylneuraminic acid or sialic acid, which is negatively charged at pH 7. Gangliosides differ from other glycolipids in that they do not incorporate such acid. They are concentrated in large amounts at the lymph node cells of the central nervous system, especially at nerve endings. Ganglosides represent 6% of the membrane of the gray matter of human brain, and, to a lesser extent, they are also found at the membranes of non-nervous animal tissues. They are located at the outer part of the membrane and perform different biological functions which are critical for the operation of the nervous system, among them the brain stabilisation and the achievement of a proper interaction between neurones and glial cells, but they also serve to recognise the cells; consequently, they are considered membrane receptors. Owing to their neurocognitive repercussion, the alteration of the metabolism of gangliosides is particularly significant. The type and abundance of gangliosides changes from one area of the brain to another, and during the different stages of brain ontogenesis.

During the seventies, some *in vitro* surveys performed on gangliosides suggested that they could promote axonal growth (Roisen 1981). The studies carried out on animals have suggested that gangliosides may exercise a protection effect on nerves and, in the long term, they may help them to grow again (Ledeen 1984). The mechanics of ganglioside action is not still clear (Blight 2002), but among those proposed we should include their anti-excitotoxic activity, prevention of apoptosis and potentiation of reinnervation by nerve sprouts and of the effects of nervous growth factors (Geisler 2001). Some clinical essays have been performed with ganglisodes for certain neurological diseases, especially cerebrovascular accidents (a systematic Cochrane review has been performed in connection with this use [Candelise 2003]) and Parkinson's Disease (Schneider 1998).

Therefore, the food product of this invention provides a nutritional supplement which is especially appropriate for the treatment of those individuals suffering neurological or neurodegenerative alterations, or for the prevention of such alterations, as well as for the prevention of cognitive deterioration conditions or behaviour disorders, especially among elderly people. The product allows a synergic metabolic activity thanks to the administration of DHA, phospholipids and phospholipid precursors (UMP, IMP, choline) and gangliosides, simultaneously facilitating, thanks to the supply of folic acid and vitamins of the B group, an adequate source for the construction of DNA and RNA components, and inhibiting neuronal apoptosis. On the other hand, inosine partially allows neuronal regeneration of the brain areas affected by infarction.

Apart from those components which have been previously mentioned, the food product of this invention also contains L-taurine (a widely known neuroprotector and inducer and modulator of synaptic plasticity) and L-carnitine (a potentiator of the brain function, which improves memory and mind acuteness in elderly people, as well as senile dementia and Alzheimer's disease) in the following amounts:

| | *Per every 100 ml of product* |
|---|---|
| L-Taurine | 8 mg |
| L-Carnitine | 8 mg |

The complete food product intended for nutritional use has been especially designed for enteral or oral nutrition of patients who suffer neurological or neurodegenerative alterations, and for the prevention of cognitive or behaviour disorders, especially in the case of elderly persons, such food product comprising a functional ingredient especially conceived for the nourishment of patients suffering from, or prone to neurological or neurodegenerative alterations, cognitive deterioration or behaviour disorders, and is supplemented with the appropriate amounts of vitamins and minerals, in line with the latest recommendations of the American Institute of Medicine (IOM 1998-2005).

Optionally, the food product of this invention also contains the customary additives for this kind of preparations intended for oral or enteral administration, such as excipients, sweeteners, stabilisers, etc., depending on its presentation, preferably in liquid form. In one embodiment, the food product of the invention is a liquid product containing flavours to enhance its palatability, preferably cocoa or vanilla flavour.

In one exemplary embodiment of the food product according to the invention, in liquid form and with cocoa taste, the product comprises the ingredients and amounts shown in Table 1 below:

**Table 1**

| **Average nutritional information** | | | | | **Per 100 ml** |
|---|---|---|---|---|---|
| **Energetic value** | | | | kcal | 125 |
| | | | | kJ | 525 |
| **Proteins (20%)** | | | | g | 6.0 |
| Caseinate | | | | g | 3.0 |
| Whey protein | | | | G | 1.5 |
| | | GMP | | G | 0.21 |
| Vegetable protein | | | | G | 1.5 |
| **Carbohydrates (45%)** | | | | g | 14.0 |
| | Of which, sugars | | | g | 0.40 |
| **Fats (35%)** | | | | g | 5.0 |
| | Of which | | | | |
| | | Saturated, of which | | g | 1.1 |
| | | | MCT | g | 0.43 |
| | | Monounsaturated | | g | 2.7 |
| | | Polyunsaturated, of which | | g | 1.2 |
| | | | Linoleic acid | g | 1.0 |
| | | | Linolenic acid | g | 0.12 |
| | | | EPA | g | 0.023 |
| | | | DHA | g | 0.047 |
| **Dietary fibre** | | | | g | 2.0 |
| **Inulin** | | | | g | 1.2 |
| **UMP and/or IMP** | | | | g | 0.02-0.10 |
| **L-Taurine** | | | | mg | 8.0 |
| **Phospholipids** | | | | mg | 39.0 |
| **Gangliosides** | | | | mg | 2.5 |
| **Sialic acid** | | | | mg | 0.88 |
| **L-Carnitine** | | | | mg | 8.0 |
| **Minerals** | | | | | |
| Calcium | | | | mg | 92 |
| Phosphorus | | | | mg | 85 |
| Potassium | | | | mg | 302 |
| Sodium | | | | mg | 86 |
| Chloride | | | | mg | 131 |
| Iron | | | | mg | 1.6 |
| Zinc | | | | mg | 0.92 |
| Copper | | | | µg | 148 |
| Iodine | | | | µg | 10 |
| Selenium | | | | µg | 5.4 |
| Magnesium | | | | mg | 28 |
| Manganese | | | | mg | 0.17 |
| Fluoride | | | | mg | 0.12 |
| Molybdenum | | | | µg | 9.3 |
| Chrome | | | | µg | 4.4 |

| **Vitamins** | | | | | |
|---|---|---|---|---|---|
| A | | | | µg | 60 |
| D | | | | µg | 3.0 |
| E | | | | mg | 3.1 |
| C | | | | mg | 6.0 |
| B1 | | | | mg | 0.10 |
| B2 | | | | mg | 0.11 |
| B3 | | | | mg | 1.3 |
| B6 | | | | mg | 0.18 |
| B9 | | | | µg | 40 |
| B12 | | | | µg | 0.24 |
| Biotin | | | | µg | 1.3 |
| Pantothenic acid | | | | mg | 0.80 |
| K | | | | µg | 8.0 |
| Choline | | | | mg | 55.0 |

In another exemplary embodiment of the food product according to the invention, in liquid form and with vanilla taste, the product comprises the ingredients and amounts shown in Table 2 below:

**Table 2**

| **Average nutritional information** | | | | | **Per 100 ml** |
|---|---|---|---|---|---|
| **Energetic value** | | | | kcal | 125 |
| | | | | kJ | 525 |
| **Proteins (20%)** | | | | g | 6.0 |
| | Caseinate | | | g | 3.0 |
| | Whey protein | | | g | 1.5 |
| | | GMP | | g | 0.21 |
| | Vegetable protein | | | g | 1.5 |
| **Carbohydrates (45%)** | | | | g | 14.0 |
| | Of which, sugars | | | g | 0.40 |
| **Fats (35%)** | | | | g | 5.0 |
| | Of which | | | | |
| | | Saturated, of which | | g | 1.1 |
| | | | MCT | g | 0.43 |
| | | Monounsaturated | | g | 2.7 |
| | | Polyunsaturated, of which | | g | 1.2 |
| | | | Linoleic acid | g | 1.0 |
| | | | Linolenic acid | g | **0.12** |
| | | | EPA | g | 0.023 |
| | | | DHA | g | 0.047 |
| **Dietary fibre** | | | | g | 1.9 |
| **Inulin** | | | | g | 1.2 |
| **UMP and/or IMP** | | | | g | 0.02-0.10 |
| **L-Taurine** | | | | mg | 8.0 |
| **Phospholipids** | | | | mg | 39.0 |
| **Gangliosides** | | | | mg | 2.5 |
| **Sialic acid** | | | | mg | 0.88 |
| **L-Carnitine** | | | | mg | 8.0 |
| **Minerals** | | | | | |
| Calcium | | | | mg | 94 |
| Phosphorus | | | | mg | 89 |
| Potassium | | | | mg | 316 |
| Sodium | | | | mg | 93 |
| Chloride | | | | mg | 131 |
| Iron | | | | mg | 1.5 |
| Zinc | | | | mg | 0.73 |
| Copper | | | | µg | 83.5 |
| Iodine | | | | µg | 8.6 |
| Selenium | | | | µg | 4.9 |
| Magnesium | | | | mg | 19.8 |
| Manganese | | | | mg | 0.15 |
| Fluoride | | | | mg | 0.10 |
| Molybdenum | | | | µg | 8.32 |
| Chrome | | | | µg | 2.06 |

| **Vitamins** | | | | | |
|---|---|---|---|---|---|
| A | | | | µg | 60 |
| D | | | | µg | 3.0 |
| E | | | | mg | 3.1 |
| C | | | | mg | 6.0 |
| B1 | | | | mg | 0.10 |
| B2 | | | | mg | 0.11 |
| B3 | | | | mg | 1.3 |
| B6 | | | | mg | 0.18 |
| B9 | | | | µg | 40 |
| B12 | | | | µg | 0.24 |
| Biotin | | | | µg | 1.3 |
| Pantothenic acid | | | | mg | 0.80 |
| K | | | | µg | 8.0 |
| Choline | | | | mg | 55.0 |

## Claims

1. A complete food product intended for oral or enteral nutrition, comprising a mixture of carbohydrates, a mixture of proteins, a mixture of lipids and dietary fibre, inulin, vitamins and minerals, **characterised in that** it includes a functional ingredient which has been especially designed for the nutrition of patients suffering from, or prone to neurological or neurodegenerative alterations, cognitive deterioration or behaviour disorders, and which is based on DHA, UMP and/or IMP, vitamin B1, vitamin B6, choline, folic acid, phospholipids, gangliosides and sialic acid.

2. A complete food product intended for oral or enteral nutrition according to claim 1, **characterised in that** the mixture of carbohydrates represents from 45 to 50% of the total energy of the product and includes dietary fibre and inulin.

3. A complete food product intended for oral or enteral nutrition according to claim 2, **characterised in that** it contains 2 g of dietary fibre /1.00 ml of product and 1.2 g of inulin /100 ml of product.

4. A complete food product intended for oral or enteral nutrition according to claim 1, **characterised in that** the mixture of proteins accounts for 15% to 20% of the total energy of the product and **in that** it contains 50% of caseinate, 25% of vegetable protein, preferably derived from peas, and 25% of milk whey proteins enriched with glycomacropeptide (approximately 0,16 g/100 ml of product).

5. A complete food product intended for oral or enteral nutrition according to claim 1, **characterised in that** the mixture of lipids accounts for 35% of the total energy of the product and it shows the following lipid profile (with respect to 100 g of fat): saturated fatty acids 20.00%, monounsaturated fatty acids 55.00%, polyunsaturated fatty acids, 25.00%.

6. A complete food product intended for oral or enteral nutrition according to claim 5, **characterised in that** the ratio between linoleic acid and linolenic acid is 8:1.

7. A complete food product intended for oral or enteral nutrition according to claim 5, **characterised in that** the EPA:DHA ratio equals 1:2.

8. A complete food product intended for oral or enteral nutrition according to claim 1, **characterised in that** the functional ingredient which has been especially designed for the nutrition of patients suffering from, or prone to neurological or neurodegenerative alterations, cognitive deterioration or behaviour disorders, and which is based on DHA, UMP and/or IMP, vitamin B1, vitamin B6, choline, folic acid, phospholipids, gangliosides and sialic acid comprises, per every 100 ml of product, 0.047 g of DHA, 20 mg of UMP (uridine monophosphate) and/or 20 mg of IMP (inosin monophosphate), 0.1 mg of Vitamin B1, 0.18 mg of Vitamin B6, 55 mg of Vitamin B7 (choline), 40 µg of Vitamin B9 (folic acid), 39 mg of phospholipids, 2.5 mg of gangliosides and 0.88 mg of sialic acid.

9. A complete food product intended for oral or enteral nutrition according to any of the preceding claims, **characterised in that** it also comprises, per every 100 ml of product, 8 mg of L-Taurine and 8 mg of L-Carnitine.

10. A complete food product intended for oral or enteral nutrition according to the preceding claims, **characterised in that** it also comprises sweeteners, stabilizers, colouring matter and/or flavours to improve its palatability.

11. A food product according to the precedent claims, intended to be used for the nutritional treatment or for the prevention of neurological or neurodegenerative, alterations, cognitive deterioration or behaviour disorders.
